(19) Europäisches Patentamt
European Patent Office
Office européen des brevets

(11) Veröffentlichungsnummer: 0 268 943
A1

(12) EUROPÄISCHE PATENTANMELDUNG

(21) Anmeldenummer: 87116697.1

(22) Anmeldetag: 12.11.87

(51) Int. Cl.4: C07C 127/22 , C07C 157/12 , A01N 47/34

(30) Priorität: 25.11.86 DE 3640175

(43) Veröffentlichungstag der Anmeldung: 01.06.88 Patentblatt 88/22

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Sirrenberg, Wilhelm, Dr.
Wuppertaler Strasse 21
D-4322 Sprockhövel(DE)
Erfinder: Becker, Benedikt, Dr.
Metzkausener Strasse 14
D-4020 Mettmann(DE)
Erfinder: Behrenz, Wolfgang, Dr.
Untergründemich 14
D-5063 Overath(DE)
Erfinder: Krehan, Ingomar, Dr.
Ludwig-Jahn-Strasse 54
D-5000 Köln 40(DE)
Erfinder: Stendel, Wilhelm, Dr.
In den Birken 55
D-5600 Wuppertal 1(DE)

# (54) Benzoyl (thio) harnstoffe.

(57) Die vorliegende Erfindung betrifft die neuen substituierten Benzoyl(thio)harnstoffe der allgemeinen Formel (I)

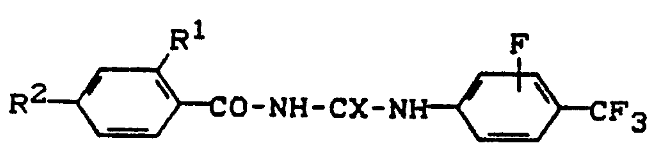

(I)

in welcher
X für Sauerstoff oder Schwefel steht,
$R^1$ für Halogen steht und
$R^2$ für Halogen steht,
welche als Schädlingsbekämpfungsmittel verwendet werden können.

EP 0 268 943 A1

## Benzoyl(thio)harnstoffe

Die vorliegende Erfindung betrifft neue Benzoyl(thio)harnstoffe, mehrere Verfahren zu ihrer Herstellung und ihre Verwendung als Schädlingsbekämpfungsmittel, insbesondere als Arthropodizide und besonders bevorzugt als Insektizide.

Es ist bereits bekannt, daß bestimmte Benzoylharnstoffe, wie z.B. 1-(3-Chlor-4-trifluormethylphenyl)-3-(2-chlor-4-fluor-benzoyl)-harnstoff insektizide Eigenschaften besitzen (vgl. z. B. EP-A 93 976).

Es wurden die neuen substituierten Benzoyl(thio)harnstoffe der allgemeinen Formel (I)

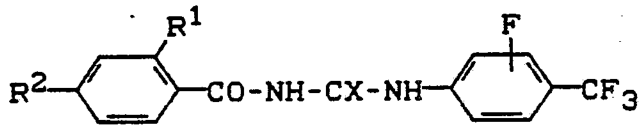 (I)

gefunden,
in welcher
X für Sauerstoff oder Schwefel steht,
$R^1$ für Halogen steht und
$R^2$ für Halogen steht.

Diese neuen Verbindungen weisen starke biologische, insbesondere arthropodizide und besonders bevorzugt insektizide Eigenschaften auf, die ihre Verwendung als Schädlingsbekämpfungsmittel, insbesondere als Insektizide ermöglichen.

Weiterin wurde gefunden, daß die neuen substituierten Benzoyl(thio)harnstoffe der allgemeinen Formel (I) erhalten werden, indem man

a) substituierte Aniline der allgemeinen Formel (II)

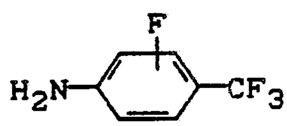 (II)

mit Benzoyl-iso(thio)cyanaten der allgemeinen Formel (III)

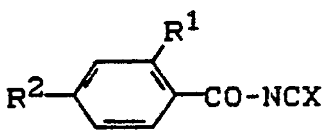 (III)

in welcher
X, $R^1$ und $R^2$ die oben angegebenen Bedeutungen haben,
gegebenenfalls in Gegenwert eines Verdünnungsmittels umsetzt oder

b) substituierte Phenyl-iso(thio)cyanate der allgemeinen Formel (IV)

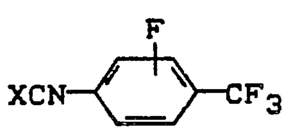 (IV)

in welcher
X die oben angegebene Bedeutung hat,
mit Benzoesäureamiden der allgemeinen Formel (V)

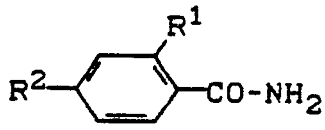

(V)

in welcher
$R^1$ und $R^2$ die oben angegebenen Bedeutungen haben,
gegebenenfalls in Gegenwart eines Katalysators und gegebenenfalls in Gegenwart eines Verdünnungsmittels umsetzt.

Halogen bedeutet in der Definition von $R^1$ und $R^2$ Fluor, Chlor, Brom und Iod, vorzugsweise Fluor, Chlor und Brom, insbesondere Fluor und Chlor.

Die neuen Verbindungen der allgemeinen Formel (I) verfügen über Eigenschaften, die ihre Verwendung als Schädlingsbekämpfungsmittel ermöglichen, insbesondere zeichnen sie sich sowohl durch eine hervorragende und langanhaltende arthropodizide, vorzugsweise insektizide Wirksamkeit aus.

Die Erfindung betrifft vorzugsweise neue Verbindungen der allgemeinen Formel (I), in welcher
X für Sauerstoff oder Schwefel (vorzugsweise Sauerstoff) steht,
$R^1$ für Fluor, Chlor oder Brom steht und
$R^2$ für Fluor, Chlor oder Brom steht.

Besonders bevorzugt sind die Verbindungen der allgemeinen Formel (I), in welcher
X für Sauerstoff oder Schwefel (vorzugsweise Sauerstoff) steht,
$R^1$ für Fluor oder Chlor steht und
$R^2$ für Fluor oder Chlor steht.

Eine besonders gute Wirksamkeit weisen Verbindungen der allgemeinen Formel (I) auf, in welcher
X für Schwefel steht,
$R^1$ für Fluor oder Chlor (vorzugsweise Chlor) steht und
$R^2$ für Fluor oder Chlor (vorzugsweise Fluor) steht,

Weiterhin sind Verbindungen der allgemeinen Formel (I) bevorzugt,
in welcher
X für Sauerstoff steht,
$R^1$ für Fluor oder Chlor (vorzugsweise Chlor) steht und
$R^2$ für Fluor oder Chlor (vorzugsweise Fluor) steht.

Ganz besonders bevorzugt werden die Verbindungen der allgemeinen Formel (I), in welcher
X für Sauerstoff steht,
$R^1$ für Chlor steht und
$R^2$ für Fluor steht.

Das Fluoratom im Phenylteil steht in der 2-oder 3-Position, vorzugsweise in der 3-Position (zur Aminogruppe).

Verwendet man gemäß Verfahrensvariante (a) 2-Chlor-4-fluorbenzoylisothiocyanat und 3-Fluor-4-trifluormethylanilin als Ausgangsstoffe, so kann der Reaktionsablauf durch das folgende Formelschema wiedergegeben werden:

a)

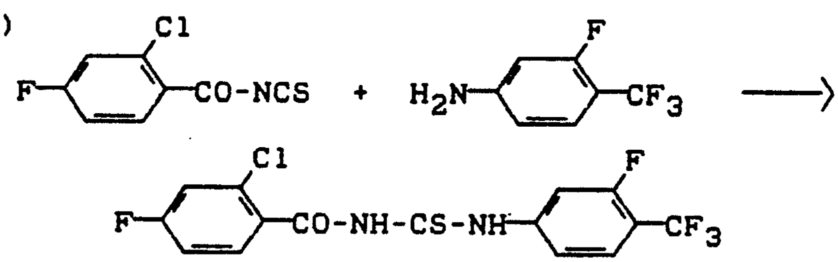

Verwendet man nach Verfahrensvariante (b) 2-Fluor-4-trifluormethyl-phenylisocyanat und 2,4-Difluorbenzamid als Ausgangsstoffe, so kann der Reaktionsablauf durch das folgende Formelschema wiedergegeben werden:

b)

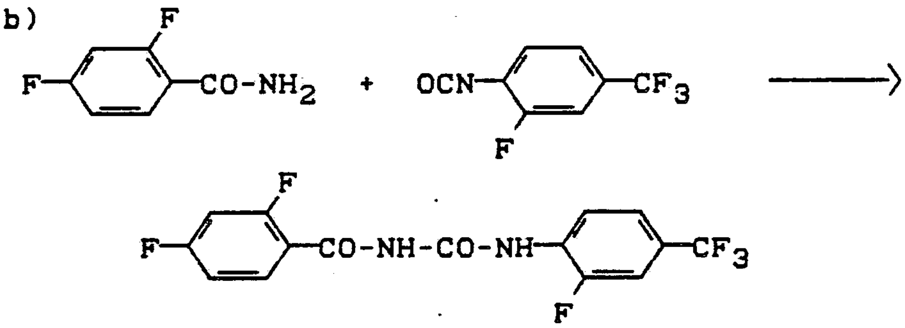

Die Ausgangsverbindungen der Formel (II) sind bekannt oder können nach allgemein bekannten Methoden erhalten werden.

Als Beispiele für die Verbindungen der Formel (II) seien genannt: 2-Fluor-und 3-Fluor-4-trifluormethyl-anilin.

Die Ausgangsverbindungen der allgemeinen Formel (III) sind bekannt oder nach allgemein bekannten Methoden erhältlich.

Als Beispiele für die Verbindungen der Formel (III) seien genannt: 2-Chlor-4-fluor-, 2,4-Difluor-und 2,4-Dichlorbenzoylisocyanat bzw. -isothiocyanat.

Die Ausgangsverbindungen der allgemeinen Formel (IV) sind bekannt oder die Aminogruppe der Verbindungen der Formel (II) kann nach üblichen Verfahren in die Isocyanat-bzw. Isothiocyanat-Gruppe umgewandelt werden, z. B. durch Umsetzung mit Phosgen bzw. Thiophosgen in Verdünnungsmitteln wie z.B. Toluol und/oder Pyridin, bei einer Temperature zwischen -20°C und +50 °C.

Als Beispiele für die Verbindungen der Formel (IV) seien genannt: 2-Fluor-und 3-Fluor-4-trifluormethyl-phenylisocyanat bzw. -phenylisothiocyanat.

Die Ausgangsverbindungen der allgemeinen Formel (V) sind ebenfalls bekannt oder nach allgemein bekannten Methoden erhältlich.

Als Beispiele für die Verbindungen der Formel (V) seien genannt: 2-Chlor-4-fluor-, 2,4-Difluor-und 2,4-Dichlorbenzoesäureamid.

Als Verdünnungsmittel kommen bei der Durchführung der Verfahrensvarianten (a) und (b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone, wie Aceton, Methylethyl-, Methylisopropyl-und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methyl-pyrrolidon, sowie Tetramethylensulfon.

Als Katalysatoren können für die Umsetzung gemäß Verfahrensvariante (b) vorzugsweise tertiäre Amine, wie Triethylamin und 1,4-Diazabicyclo-[2,2,2]-octan, sowie organische Zinn-Verbindungen, wie z.B. Dibutylzinndilaurat verwendet werden. Der Zusatz solcher Katalysatoren ist jedoch nicht unbedingt erforderlich.

Die Reaktionstemperatur kann bei den Verfahrensvarianten (a) und (b) innerhalb eines größeren Bereiches variiert werden. Im allgemeinen arbeitet man bei der Verfahrens variante (a) zwischen 20 °C und 180 °C, vorzugsweise zwischen 40 °C und 120 °C und bei der Verfahrensvariante (b) zwischen 20 °C und 200 °C, vorzugsweise zwischen 60 °C und 190 °C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponenten bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierungdient der Schmelzpunkt.

Die Wirkstoffe der allgemeinen Formel (I) eignen sich bei guter Pflanzenverträglichkeit und günstiger Warmblütertoxizität zur Bekämpfung von tierischen Schädlingen, vorzugsweise Arthropoden, insbesondere Insekten, die in der Landwirtschaft, im Gartenbau, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blanius guttulatus.
Aus der Ordnung der Chilopoda z.B. Geophilus carpophagus, Scutigera spec..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ornung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Erisoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z.B. Pectinophera gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenes spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstritialis, Cotelytra zealandica.
Aus der Ordnung der Hymenoptera z.B. Diprion spp., Hoplocampa spp., Lasius spp., Monomorium pharaonis, Vespa spp..
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xeropsylla cheopis, Ceratophyllus spp.,
Aus der Ordnung der Arachnida z.B. Scorpio maurus, Latrodectus mactans.

Die erfindungsgemäßen Wirkstoffe der Formel (I) zeichnen sich insbesondere durch eine hervorragende insektizide Wirksamkeit aus. Sie zeigen beim Einsatz als Insektizide eine hervorragende Wirkung und eine besonders lange Wirksamkeit vorzugsweise gegen Käferlarven, wie z. B. Phaedon cochleariae und Raupen (insbesondere von Schmetterlingen)wie z. B. Spodoptera frugiperda. Die neuen Verbindungen zeigen außerdem eine ausgezeichnete Wirkung bei der Bekämpfung von Lästlingen oder Hygieneschädlingen wie Mückenlarven und Fliegenmaden, wie Musca (insbesondere Musca domestica) oder Aedes (insbesondere Aedes aegypti).

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellts, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe,wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polar Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und orgnischen Mehlen sowie Granulate aus organischem Material wie Sägemehl, Kokosnußschalen, Maiskolben und Tabakstengel; als Emulgier-und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglycol-Ether, Alkysulfonate, Alkysulfonate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mine-ralische und vegetabile Öle sein.

Es können Farbstoffe wie anorganische Pigmente, z. B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeine zwischen 0,1 und 95 Gewichtsprozent Wirkstoff, vorzugsweise zwischen 0,5 und 90 %.

Die erfindungsgemäßen Wirkstoffe können in ihren handelsüblichenFormulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe der Formel (I) eignen sich auch zur Bekämpfung von Arthropoden, die im Zusammenhang mit landwirtschaftlichen Nutztieren, wie z.B. Rinder, Schafe, Ziegen, Pferde, Schweine, Esel, Kamele, Büffel, Kaninchen, Hühner, Puten, Enten, Gänse oder sonstigen Hustieren wie z.B. Hunde, Katzen, Stubenvögel, Aquarienfische sowie sogenannten Versuchstieren, wie z.B. Hamster, Meerschweinchen, Ratten und Mäuse, auftreten können. Durch die Bekämpfung dieser Arthropoden sollen Todesfälle und Leistungsminderungen (bei Fleisch, Milch, Wolle, Häuten, Eiern usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht in diesen Bereichen, z.B. nach den üblichen Methoden (wie sie von Benzoylharnstoffen bekannt sind) z.B. durch oberflächige Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie

Halsbändern, Ohrmarken, Schwanzmarken, Gliedmaßenbändern, Halftern, Markierungsvorrichtungen usw.

Die Wirksamkeit der erfindungsgemäßen Verbindungen der allgemeinen Formel (I) soll anhand der folgenden biologischen Beispiele erläutert werden:

Beispiel A

Phaedon-Larven-Test

Lösungsmittel: 15 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 /5 bedeutet, daß keine Käfer-Larven abgetötet wurden.

Bei diesem Test zeigte z.B> bei einer Wirkstoffkonzentration von 0,0001 % die Verbindung des Herstellungsbeispiels (2) nach 10 Tagen eine Abtötung von 100 %.

Beispiel B

Spodoptera-Test

Lösungsmittel: 15 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. bei einer Wirkstoffkonzentration von 0,00001 % die Verbindungen der Herstellungsbeispiele (1) und (2) nach 7 Tagen eine Abtötung von 100 %.

Beispiel C

Mückenlarven-Test

Testtiere: 2. Larvenstadtium Aedes aegypti
Lösungsmittel: 99 Gewichtsteile Aceton
Emulgator: 1 Gewichtsteil Benzylhydroxydiphenylpolyglykolether

Zur Herstellung einer zweckmäßigen Wirkstoffzubereitung löst man 2 Gew.-Teile Wirkstoff in 1000 Volumenteilen Lösungsmittel, das Emulgator in der oben angegebenen Menge enthält. Dis so erhaltene Lösung wird mit Wasser auf die gewünschten geringeren Konzentrationen verdünnt.

Man füllt die wäßrigen Wirkstoffzubereitungen der gewünschten Konzentration in Kunstoffbecher und setzt ansschließend 20 Mückenlarven in jeden Becher ein. Die Larven werden täglich mit Fischfutter (Tetramin®) gefüttert.

Nach $1^d$ , $8^d$ und $21^d$ wird die Abtötung in % bestimmt . Dabei bedeutet 100 %, daß alle Larven abgetötet worden sind. 0 % bedeutet, daß überhaupt keine Larven abgetötet worden sind.

Bei diesem Test zeigte z.B. bei einer Wirkstoffkonzentration von $10^{-3}$ ppm die Verbindung des Herstellungsbeispiels (2) nach 8 Tagen eine Abtötung von 100 %.

Beispiel D

$LD_{100}$-Test

Testtiere: Musca domestica (resistent)-Maden
Zahl der Testtiere; 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentration verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

14 Tage nach dem Ansetzen der Versuche wird die Zahl der geschlüpften Fliegen getestet und nach Abbott der Wirkungsgrad in % ermittelt. Dabei bedeuten 0 %, das alle Testtiere geschlüpft sind und 100 %, daß keine Testtiere geschlüpft sind.

Bei diesem Test zeigten z.B. bei eiener Wirkstoffkonzentration von 0,002 % die Verbindungen der Herstellungsbeispiele (1) und (4) eine Schlüpfverhinderung von 100 %.

Beispiel E

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigte z. B. bei einer Wirkstoffkonzentration von 100 ppm die Verbindung des Herstellungsbeispiels (2) eine Abtötung von 100 %.

Die Herstellung der erfindungsgemäßen Verbindungen soll durch die folgenden Herstellungsbeispiele erläutert werden.

Beispiel 1

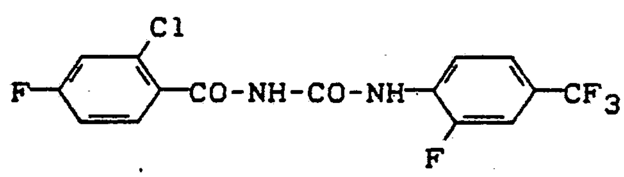

(Verfahrensvariante (a))

Zu einer Lösung von 1,79 g (0,01 Mol) 2-Fluor-4-trifluormethyl-anilin in 40 ml trockenem Toluol wird eine Lösung von 2,0 g (0,01 Mol) 2-Chlor-4-fluorbenzoylisocyanat in 10 ml trockenem Toluol gegeben, eine halbe Stunde bei 80 °C gerührt und anschließend im Vakuum eingeengt. Der Rückstand wird mit wenig Toluol und Petrolether gewaschen und anschließend getrocknet.

Man erhält 3,4 g (90 % der Theorie) 1-(2-Chlor-4-fluorbenzoyl)-3-(2-fluor-4-trifluormethyl-phenyl)-harnstoff vom Schmelzpunkt 194 °C.

Beispiel 2

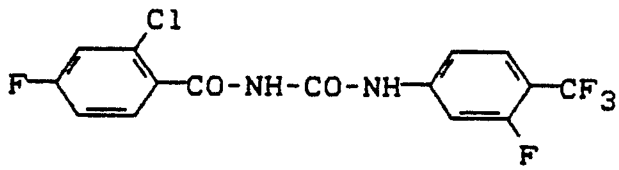

(Verfahrensvariante (b))

Zu einer Lösung von 1,74 g (0,01 Mol) 2-Chlor-4-fluorbenzamid in 40 ml trockenem Toluol wird bei 60 °C eine Lösung von 2,05 g (0,01 Mol) 3-Fluor-4-trifluormethylphenylisocyanat in 10 ml trockenem Toluol gegeben und 7 Stunden unter Rückfluß erhitzt. Anschließend wird das Reaktionsgemisch im Vakuum eingeengt. Der Rückstand wird mit wenig kaltem Diethylether verrührt, abgetrennt und getrocknet.

Man erhält 3,2 g (84,5 % der Theorie) 1-(2-Chlor-4-fluorbenzoyl)-3-(-3-fluor-4-trifluormethyl-phenyl)-harnstoff vom Schmelzpunkt 181 °C.

Analog Beispiel 1 und 2 bzw. Verfahrensvariante (a) oder (b) können die in der nachfolgenden Tabelle 1 aufgeführten Verbindungen der Formel (I) hergestellt werden:

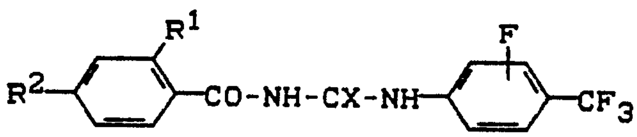 (I)

**Tabelle 1**

| Beisp.-Nr. | $R^1$ | $R^2$ | X | 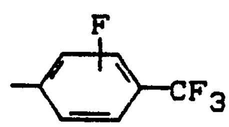 | Schmelzpunkt /[° C] |
|---|---|---|---|---|---|
| 3 | F | F | O | 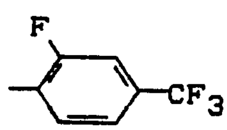 | 208 |
| 4 | Cl | F | S | 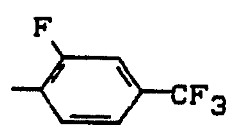 | 176 |
| 5 | Cl | F | S | 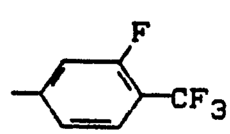 | 139 |
| 6 | F | F | O | 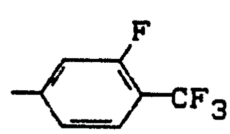 | 182 |

## Ansprüche

1. Benzoyl(thio)harnstoffe der allgemeinen Formel (I)

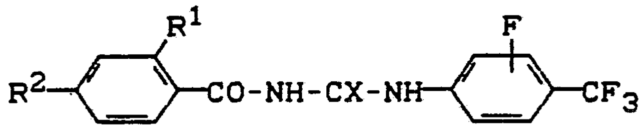

(I)

in welcher
X für Sauerstoff oder Schwefel steht,
$R^1$ für Halogen steht und
$R^2$ für Halogen steht.

2. Verbindungen der allgemeinen Formel (I) gemäß Anspruch 1, in welcher
X für Sauerstoff oder Schwefel steht,
$R^1$ für Fluor, Chlor oder Brom steht und
$R^2$ für Fluor oder Brom steht.

3. Verbindungen der allgemeinen Formel (I) gemäß Anspruch 1, in welcher
X für Sauerstoff oder Schwefel steht,
$R^1$ für Fluor oder Chlor steht und
$R^2$ für Fluor oder Chlor steht.

4. Verbindung der Formel

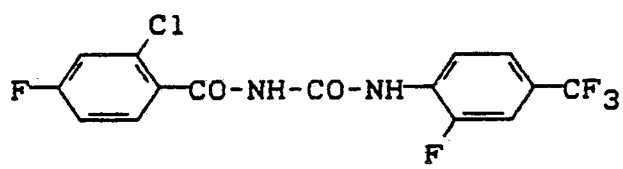

5. Verbindung der Formel

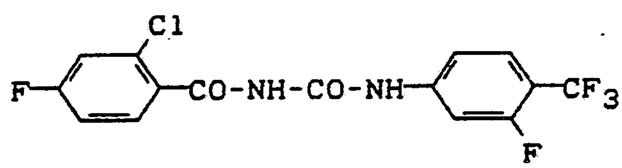

6. Verfahren zur Herstellung die Benzoyl(thio)harnstoffe der allgemeinen Formel

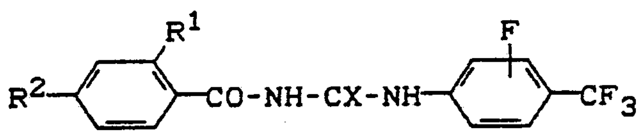

(I)

in welcher
X für Sauerstoff oder Schwefel steht,
$R^1$ für Halogen steht und
$R^2$ für Halogen steht,
dadurch gekennzeichnet, daß man

a) substituierte Aniline der allgemeinen Formel (II)

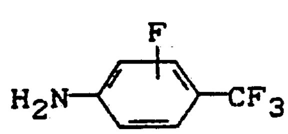

(II)

mit Benzoyl-iso(thio)cyanaten der allgemeinen Formel (III)

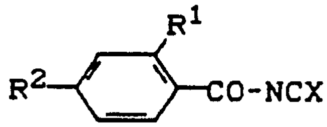

(III)

in welcher
X, $R^1$ und $R^2$ die oben angegebenen Bedeutungen haben, gegebenenfalls in Gegenwart eines Verdünnungsmittels umsetzt oder

b) substituierte Phenyl-iso(thio)cyanate der allgemeinen Formel (IV)

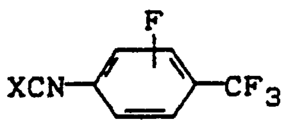

(IV)

in welcher
X die oben angegebene Bedeutung hat, mit Benzoesäureamiden der allgemeinen Formel (V)

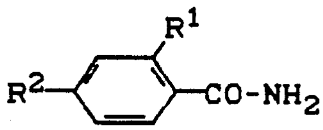

(V)

in welcher
$R^1$ und $R^2$ die oben angegebenen Bedeutungen haben, gegebenenfalls in Gegenwart eines Katalysators und gegebenenfalls eines Verdünnungsmittels umsetzt.

7. Schädlingsbekämpfungsmittel, gekennzeichnet durch einen Gehalt an mindestens einer Verbindung gemäß den Ansprüchen 1 bis 5 bzw. mindestens einer Verbindung der Formel (I) gemäß Anspruch 6.

8. Verwendung von Verbindungen gemäß den Ansprüchen 1 bis 5 bzw. mindestens einer Verbindung der Formel (I) gemäß Anspruch 6 zur Bekämpfung von Schädlingen, insbesondere von Insekten.

9. Verfahren zur Bekämpfung von Schädlingen, dadurch gekennzeichnet, daß man wenigstens eine Verbindung gemäß den Ansprüchen 1 bis 5 bzw. mindestens eine Verbindung der Formel (I) gemäß Anspruch 6 auf die Schädlinge, vorzugsweise Insekten oder ihren Lebensraum einwirken läßt.

10. Verfahren zur Herstellung von Schädlingsbekämpfungsmitteln, dadurch gekennzeichnet, daß man Verbindungen gemäß den Ansprüchen 1 bis 5 bzw. Verbindungen der Formel (I) gemäß Anspruch 6 mit Streckmitteln und/oder oberflächenaktiven Mitteln vermischt.

Europäisches Patentamt

# EUROPÄISCHER RECHERCHENBERICHT

Nummer der Anmeldung

EP 87 11 6697

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 531 279 (BAYER)<br>* Anspruch 1 *<br>--- | 1 | C 07 C 127/22<br>C 07 C 157/12<br>A 01 N 47/34 |
| A,D | EP-A-0 093 976 (BAYER)<br>* Seite 28, Beispiele 7,19 *<br>----- | 1 | |

| RECHERCHIERTE SACHGEBIETE (Int. Cl.4) |
|---|
| C 07 C 127/22<br>C 07 C 157/12<br>A 01 N 47/34 |

Der vorliegende Recherchenbericht wurde für alle Patentansprüche erstellt

| Recherchenort | Abschlußdatum der Recherche | Prüfer |
|---|---|---|
| BERLIN | 19-01-1988 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)